# EUROPÄISCHE PATENTSCHRIFT

(11) **EP 2 011 483 B1**
(45) Veröffentlichungstag und Bekanntmachung des Hinweises auf die Patenterteilung: **23.03.2011**
(21) Anmeldenummer: 08104171.7
(22) Anmeldetag: 30.05.2008
(51) Int. Cl.: A61K 8/92, A61Q 19/00

(54) **Kosmetische oder Pharmazeutische Zubereitung**
Pharmaceutical or cosmetic preparation
Préparation cosmétique ou pharmaceutique

(30) Priorität: 06.07.2007 DE 102007031697
(43) Veröffentlichungstag der Anmeldung: 07.01.2009
(73) Patentinhaber: Walter RAU Neusser Öl und Fett AG, 41460 Neuss (DE)
(72) Erfinder: Brinkmann, Bernd, 41812, Erkelenz (DE)
(74) Vertreter: Bauer, Dirk

(56) Entgegenhaltungen:
- WO-A1-00/69273
- WO-A1-2007/103398
- DE-A1- 2 654 743
- DE-U1-202005 019 453
- DE-U1-202005 019 454
- US-A1- 2003 207 971
- US-A1- 2006 257 353

## Beschreibung

### Einleitung

Die Erfindung betrifft eine kosmetische oder pharmazeutische Zubereitung, wie sie typischerweise in Cremes, Hautlotionen, Lippenstiften und anderen kosmetischen Endprodukten oder in Salben im Bereich der Pharmazie zur Anwendung kommt. Insbesondere betrifft die Erfindung eine auf rein pflanzlicher Basis hergestellte Zubereitung, die als Ersatzstoff bzw. Austauschstoff für die bekannte Vaseline (Petrolatum) dienen soll.

### Stand der Technik

Bei der im Kosmetik- und Pharmaziebereich häufig verwendeten und seit geraumer Zeit allgemein bekannten Vaseline handelt es sich um ein Produkt auf Erdölbasis. Gerade im Kosmetikbereich stehen Inhaltsstoffe auf Basis von Erdölprodukten wegen möglicherweise nachteiliger Eigenschaften häufig in der Kritik. Insbesondere die Pflegeeigenschaften von Erdölderivaten sind aufgrund der die Porengröße menschlicher Haut übersteigenden Molekülgröße bestimmter Komponenten der Vaseline schlechter als die von Stoffen insbesondere pflanzlicher Herkunft.

Aufgrund dieser Kritik ist bereits versucht worden, einen Esatzstoff für Vaseline auf pflanzlicher Basis zu schaffen. So beschreiben die deutschen Gebrauchsmuster DE 20 2005 019 453 U1 und DE 20 2005 019 454 U1 jeweils pflanzliche kosmetische Erzeugnisse, die aus Pflanzenölen, Pflanzenfetten, Pflanzenwachsen und möglicherweise Bienenwachs, Vitamin E und Duftstoffen, insbesondere ätherischen Ölen, bestehen. Nach den beiden vorgenannten Gebrauchsmusterschriften werden zur Herstellung so genannter veganer Bio-Melkfette insbesondere die nachfolgenden Pflanzenöle bzw. □fette verwendet: Rapsöl, Palmkernöl, Olivenöl, Rizinusöl, Jojobaöl, Palmsterin Sheabutter und Kokosfett.

Als nachteilig tritt bei der bekannten Zubereitung in Erscheinung, dass die Einstellung der gewünschten Viskosität aufgrund der Verwendung von Naturprodukten mit typischerweise schwankender Zusammensetzung schwierig ist. Außerdem können die typischen rheologischen Eigenschaften der Vaseline nicht in dem gewünschten Maß erreicht werden. Insbesondere ist die Haltbarkeit der vorbekannten Zubereitung nicht zufrieden stellend; nach gewisser Lagerzeit, besonders bei höheren Temperaturen, können bestimmte Komponenten in der vorbekannten Zubereitung ranzig werden.

### Aufgabe

Der Erfindung liegt die Aufgabe zugrunde, eine kosmetische oder pharmazeutische Zubereitung auf pflanzlicher Grundlage bereitzustellen, die die Eigenschaften von Vaseline, insbesondere deren rheologischen Eigenschaften, die Hautschutzeigenschaften sowie die lange Haltbarkeit möglichst gut imitiert.

### Lösung

Erfindungsgemäß wird die vorgenannte Aufgabe durch eine kosmetische oder pharmazeutische Zubereitung gelöst, die
- 60 % bis 98 % einer Mischung mittelkettiger Triglyceride (MCT)
- 2 % bis 40 % einer Mischung langkettiger Triglyceride (LCT)
enthält. Vorzugsweise liegt der Gehalt an MCT-Mischung zwischen 70 % und 95 % und der Gehalt an LCT-Mischung zwischen 5 % und 30 %. Weitere Komponenten können insbesondere von Ölen, Fetten und Wachsen, insbesondere solcher pflanzlicher Herkunft, gebildet werden. Bei sämtlichen %-Angaben in dieser Anmeldung handelt es sich um Gewichtsprozente (Gew.-%).

Die erfindungsgemäß verwendeten MCT und LCT sollten vollständig aus pflanzlichen Rohstoffen gewonnen sein. Definitionsgemäß sollte das mittelkettige Triglycerid-Gemisch Fettsäuren mit Kettenlängen von 8 bis 10 Kohlenstoffatomen (C8 bis C10) aufweisen. Das langkettige Triglycerid-Gemisch sollte dem gegenüber vorzugsweise aus Fettsäuren mit Kettenlängen von C18 bis C24 bestehen.

In besonders vorteilhafter Weise sollte die LCT-Mischung einen Gehalt an Fettsäuren mit einer Kettenlänge der Kohlenstoffatome größer als bzw. gleich 20 von mehr als 10 %, weiter vorzugsweise mehr als 40 % und noch weiter vorzugsweise mehr als 50 % aufweisen.

Aus Gründen einer möglichst langen Haltbarkeit der erfindungsgemäßen Zubereitung sollte es sich bei den Fettsäuren sowohl der MCT-Mischung als auch der LCT-Mischung um gesättigte Fettsäuren handeln. Im Falle ungesättigter Fettsäuren, wie diese bei natürlichen Ölen und Fetten häufig vorkommen, kann bedarfsweise eine □teilweise oder vollständige □Hydrierung (Härtung) erfolgen. Die Gefahr eines Ranzigwerdens der erfindungsgemäßen Zubereitung wird somit ausgeräumt.

Die Jodzahl, der verwendeten Öle bzw. Fette sollte zwischen 0 und 30 liegen, vorzugsweise zwischen 0 und 2, weiter vorzugsweise zwischen 0 und 1.

Als Ausgangsstoff für die langkettige Triglyceridmischung ist insbesondere ein Rapsöl mit einem hohen Gehalt an Erucasäure, einer einfach ungesättigten Fettsäure mit 22 Kohlenstoffatomen, sinnvoll. Bevorzugt ist dabei die Verwendung der so genannten Behensäure, bei der es sich um die ausgehärtete Form der Erucasäure handelt. Die Behensäureglyceride führen zu sehr vorteilhaften Erstarrungseigenschaften der Zubereitung. Bereits bei sehr niedrigen Anteilen an der Zubereitung insgesamt in der Größenordnung zwischen 1 % und 2,5 %, vorzugsweise ca. zwischen 2,5 % und 15 %, wirkt die Behensäure als Gerüstbildner für eine sehr gute Kristallstruktur.

Besonders bevorzugt ist es dabei, wenn die Behensäure in der Zubereitung in der so genannten β'-Form der Kristallstruktur vorliegt. Diese Kristallstruktur zeichnet sich durch besonders feine Kristalle und ein dadurch bedingtes sehr großes Ölbindevermögen aus. Der Gehalt an LCT-Mischung an der Zubereitung kann auf diese Weise niedrig gehalten werden. Außerdem wird hierdurch bei der Zubereitung gemäß der Erfindung die für Vaseline typische rheologische Eigenschaft erzielt, dass die Viskosität im mechanisch nicht belasteten Zustand der Zubereitung hoch ist, wohingegen sie unter mechanischer Belastung, d.h. insbesondere unter Scherbeanspruchung, absinkt, so dass die Zubereitung nach Art einer Creme verrieben und auf den betreffenden Partien der Haut verteilt werden kann. Nach dem Applizieren hat die Zubereitung dann wieder eine ausreichende Standfestigkeit, d.h. es findet auch bei Körpertemperatur kein unerwünschtes Verlaufen statt, was bei einer Vielzahl kosmetischer oder pharmazeutischer Anwendungsfälle von Bedeutung ist.

Für die LCT-Mischung können im Rahmen der Erfindung auch umgeesterte Mischungen aus Pflanzenfetten verwendet werden, insbesondere wenn ein niedriger Schmelzpunkt erwünscht ist. In diesem Fall kommen gehärtete oder ungehärtete Kokosöle in Betracht.

Auch wenn Triglyceride auf Basis von Behensäure für die LCT-Mischung bevorzugt werden, ist grundsätzlich auch die Verwendung anderer Pflanzenöle, insbesondere in vollhydrierter Form denkbar. So sind grundsätzlich auch vollhydrierte Rapsöle, vollhydrierte Sonnenblumenöle oder vollhydriertes Sojaöl einsetzbar. Die in Frage kommenden Öle sollten hohe bzw. überwiegende Anteile an Stearinsäure (C18) und/oder Arachinsäure (C20) enthalten. Die β-Form der Kristallstruktur ist aufgrund der gröberen Kristalle grundsätzlich nicht so erwünscht, wie die bei den vorgenannten C22-Fettsäuren genannte β'-Form, diese kann aber durch gezielte Umesterung erreicht werden.

Für die Herstellung der MCT-Mischung bietet sich insbesondere Kokosöl an, das einen vergleichsweise hohen Gehalt an Caprylsäure (C8) und Caprinsäure (C10) besitzt. Diese MCT-Mischungen haben eine sehr hohe Oxidationsstabilität, da sie ausschließlich aus den fraktionierten gesättigten Fettsäuren bestehen.

Bei Raumtemperatur liegt der Gehalt an auskristallisierten Fetten (Solid-Fat-Content (SCF)) der Zubereitung ungefähr zwischen 1 % und 40 %, vorzugsweise zwischen 5 % und 30 %.

Um die Gebrauchseigenschaften der erfindungsgemäßen Zubereitung wunschgemäß einstellen zu können, können weitere Komponenten aus Wachsen (insbesondere pflanzlichen Wachsen, aber auch Bienenwachs) sowie weiteren Ölen mit aktiven Eigenschaften, auch ätherische Öle, bestehen.

Die erfindungsgemäße Zubereitung ist typischerweise wasserfrei. Sie kann jedoch durchaus auch als Lipidphase einer Emulsion dienen, die dann das fertige kosmetische oder pharmazeutische Produkt bildet bzw. zu dessen Herstellung verwendet wird. Bei der Zubereitung kann es sich somit sowohl um ein Endprodukt als auch um ein Halbfabrikat, d.h. Rohstoff, zur weiteren Verwendung in der Kosmetik- oder Pharmaziebranche, handeln.

### Ausführungsbeispiele

Die Erfindung wird nachfolgend anhand von Beispielen für Zubereitungen in Form jeweils einer pflanzlichen Vaseline näher erläutert:

### Beispiel 1:

Die Zubereitung besteht aus einer Mischung aus 88 % einer MCT-Mischung, die als Trägeröl dient, und 6 % einer LCT-Mischung, die bei Raumtemperatur in der Zubereitung in Form einer β'-Kristallform mit feinen Kristallen und hohem Ölbindevermögen vorliegt. Darüber hinaus enthält die Zubereitung 6 % Bienenwachs, das auf der Haut eine mechanisch stabile wasserunlösliche Schicht bildet und somit eine Barrierefunktion entfaltet, wie sie auch klassische Vaseline auf Erdölbasis besitzt.

Die MCT-Mischung der beispielhaften Zubereitung besteht aus ca. 60 % C8- und 40 % C10-Fettsäuren, die Jodzahl der MCT-Mischung ist kleiner 1.

Die LCT-Mischung enthält im vorliegenden Fall ca. 50 % Behensäure aus gehärtetem erucasäurereichem Rapsöl. Die LCT-Mischung enthält des Weiteren ca. 40 % Stearinsäure (C18) und in kleinen Anteilen von insgesamt ca. 10 % andere Fettsäuren. Die Kristalle der Behensäureglyceride dienen als Gerüstbildner beim Aufbau einer unter physiologischen Gesichtspunkten betrachtet sehr angenehmen Kristallstruktur der erfindungsgemäßen Zubereitung.

Der Festfettgehalt der wasserfreien beispielhaften Zubereitung beträgt 10 % bei 20°C.

### Beispiel 2:

Die Zubereitung besteht aus einer Mischung aus 90 % einer MCT-Mischung, die als Trägeröl dient, und 10 % einer LCT-Mischung, die bei Raumtemperatur in der Zubereitung in Form einer β'-Kristallform mit feinen Kristallen und hohem Ölbindevermögen vorliegt.

Die MCT-Mischung der beispielhaften Zubereitung besteht aus ca. 60 % C8- und 40 % C10-Fettsäuren, die Jodzahl ist kleiner 1.

Die LCT-Mischung enthält im vorliegenden Fall ca. 50 % Behensäure aus gehärtetem erucasäurereichem Rapsöl. Die LCT-Mischung enthält des Weiteren ca. 40 % Stearinsäure (C18) und in kleinen Anteilen von insgesamt ca. 10 % andere Fettsäuren. Die Kristalle der Behensäureglyceride dienen als Gerüstbildner beim Aufbau einer unter physiologischen Gesichtspunkten betrachtet sehr angenehmen Kristallstruktur der erfindungsgemäßen Zubereitung.

Der Fest-Fettgehalt der wasserfreien beispielhaften Zubereitung beträgt 9 % bei 20°C. Optisch und rheologisch entspricht dieses Beispiel einer handelsüblichen Vaseline.

### Alternative Rezeptur:

Eine Zubereitung besteht im Falle einer nicht erfindungsgemäßen Rezeptur aus einer Mischung aus 99 % einer MCT-Mischung, die als Trägeröl dient, und 1 % einer LCT-Mischung, die bei Raumtemperatur in der Zubereitung in Form einer β'-Kristallform mit feinen Kristallen und hohem Ölbindevermögen vorliegt. Die MCT-Mischung der beispielhaften Zubereitung besteht aus ca. 60 % C8- und 40 % C10-Fettsäure, die Jodzahl ist kleiner 1.

Die LCT-Mischung enthält im vorliegenden Fall ca. 50 % Behensäure aus gehärtetem erucasäurereichem Rapsöl. Die LCT-Mischung enthält des Weiteren ca. 40 % Stearinsäure (C18) und in kleinen Anteilen von insgesamt ca. 10 % andere Fettsäuren. Die Kristalle der Behensäureglyceride dienen als Gerüstbildner beim Aufbau einer Kristallstruktur.

Der Fest-Fettgehalt der wasserfreien beispielhaften Zubereitung bei 20°C wurde mit < 1% bestimmt. Dieses Muster sieht trüb aus und verfestigt sich nicht, da der erfindungsgemäße Geltungsbereich verlassen wurde.

Die Überprüfung der rheologischen Eigenschaften erfolgte in allen drei vorgenannten Fällen mittels eines handelsüblichen Rheometers. Bestimmt wurde das Speichermodel G' in [Pa], bei 37 °C. Die Proben wurden zunächst 5 Minuten. oszillativ beansprucht, anschließend wurde nach 10 Minuten der Strukturaufbau gemessen (Tabelle 1). Die erfindungsgemäße Rezeptur zeichnet sich durch rheologische Eigenschaften aus, die im Bereich handelsüblicher Vaseline liegen. Ein Abweichen von der Rezeptur der erfindungsgemäßen Zubereitung führt entweder zu viel zu weichen oder zu harten Produkten.

Bei einer Lagerung bei Raumtemperatur waren die in den Beispielen 1 und 2 beschriebenen Zubereitungen über einen Zeitraum von 12 Monaten völlig oxidationsstabil, d.h. zeigten keinerlei Tendenz zum Ranzigwerden.

**Tabelle 1:**

| Probe | Zusammensetzung | G' in [Pa] bei 37 °C |
|---|---|---|
| Handelsmuster 1 (Supermarkt) | 100 % Paraffine | 670 |
| Handelsmuster 2 (Drogerie) | 100 % Paraffine | 1250 |
| Handelsmuster 3 (Apotheke) | 100 % Paraffine | 2800 |
| Beispiel 1 | 88 % MCT, 6 % LCT, | 2200 |
| | 6 % Bienenwachs | |
| Beispiel 2 | 90 % MCT, 10 % LCT | 2000 |
| nicht erfindungsgemäße Rezeptur | 99 % MCT, 1 % LCT | < 10 |

## Patentansprüche

1. Kosmetische oder pharmazeutische Zubereitung enthaltend
- 60 % bis 98 % einer Mischung aus mittelkettigen Triglyceriden (MCT)
- 2 % bis 40 % einer Mischung aus langkettigen Triglyceriden (LCT)

2. Zubereitung nach Anspruch 1 enthaltend
- 70 % bis 95 % einer Mischung aus mittelkettigen Triglyceriden (MCT)
- 5 % bis 30 % einer Mischung aus langkettigen Triglyceriden (LCT)

3. Zubereitung nach Anspruch 1 oder 2, **dadurch gekennzeichnet, dass** die LCT-Mischung einen Gehalt an Fettsäuren mit einer Kettenlänge der Kohlenstoffatome größer als oder gleich 20 zwischen 10 % und 100 %, vorzugsweise zwischen 40 % und 70 %, weiter vorzugsweise ca. 50 % aufweist.

4. Zubereitung nach einem der Ansprüche 1 bis 3, **gekennzeichnet durch** einen Gehalt an Behensäure von insgesamt mindestens 1 %, vorzugsweise mindestens 2,5 %, und höchstens 15 %.

5. Zubereitung nach Anspruch 4, **dadurch gekennzeichnet, dass** die Behensäureglyceride enthaltende LCT-Mischung in der Zubereitung bei Raumtemperatur in der β'-Form der Kristallstruktur vorliegt.

6. Zubereitung nach einem der Ansprüche 1 bis 5, **dadurch gekennzeichnet, dass** der Gehalt an Caprylsäure enthaltender MCT-Mischung in der Zubereitung zwischen 50 % und 65 % liegt.

7. Zubereitung nach einem der Ansprüche 1 bis 6, **dadurch gekennzeichnet, dass** der Gehalt an Caprinsäure enthaltender MCT-Mischung in der Zubereitung zwischen 30 % und 45 % liegt.

8. Zubereitung nach einem der Ansprüche 1 bis 7, **dadurch gekennzeichnet, dass** die Jodzahl der MCT-Mischung und/oder der LCT-Mischung und/oder der Zubereitung insgesamt kleiner als 30, vorzugsweise kleiner als 2, weiter vorzugsweise kleiner als 1 ist.

9. Zubereitung nach einem der Ansprüche 1 bis 8, **dadurch gekennzeichnet, dass** sie wasserfrei ist.

10. Zubereitung nach einem der Ansprüche 1 bis 9, **dadurch gekennzeichnet, dass** die frei von Emulgatoren ist.

## Claims

1. A cosmetic or pharmaceutical preparation, containing
- 60 % to 98 % of a mixture of medium-chain triglycerides (MCTs)
- 2 % to 40 % of a mixture of long-chain triglycerides (LCTs).

2. The preparation according to claim 1, containing
- 70 % to 95 % of a mixture of medium-chain triglycerides (MCTs)
- 5 % to 30 % of a mixture of long-chain triglycerides (LCTs).

3. The preparation according to either claim 1 or claim 2, **characterised in that** the LCT mixture comprises a content of fatty acids with a chain length of carbon atoms greater than or equal to 20 between 10 % and 100 %, preferably between 40 % and 70%, more preferably approximately 50 %.

4. The preparation according to any one of claims 1 to 3, **characterised by** a content of behenic acid of, in total, at least 1 %, preferably at least 2.5 % and at most 15 %.

5. The preparation according to claim 4, **characterised in that** the behenic acid glyceride-containing LCT mixture is present in the preparation at ambient temperature in the β'-form of the crystal structure.

6. The preparation according to any one of claims 1 to 5, **characterised in that** the content of caprylic acid-containing MCT mixture in the preparation is between 50 % and 65 %.

7. The preparation according to any one of claims 1 to 6, **characterised in that** the content of caprylic acid-containing MCT mixture in the preparation is between 30 % and 45 %.

8. The preparation according to any one of claims 1 to 7, **characterised in that** the iodine number of the MCT mixture and/or of the LCT mixture and/or of the preparation is, in total, less than 30, preferably less than 2, more preferably less than 1.

9. The preparation according to any one of claims 1 to 8, **characterised in that** it is anhydrous.

10. The preparation according to any one of claims 1 to 9, **characterised in that** it is free from emulsifiers.

## Revendications

1. Préparation cosmétique ou pharmaceutique contenant
- de 60 % à 98 % d'un mélange de triglycérides à chaîne moyenne (MCT)
- de 2 % à 40 % d'un mélange de triglycérides à chaîne longue (LCT).

2. Préparation selon la revendication 1 contenant
- de 70 % à 95 % d'un mélange de triglycérides à chaîne moyenne (MCT)
- de 5 % à 30 % d'un mélange de triglycérides à chaîne longue (LCT).

3. Préparation selon la revendication 1 ou 2, **caractérisée en ce que** le mélange de LCT comporte une teneur comprise entre 10 % et 100 %, de préférence entre 40 % et 70 %, de manière encore plus préférée d'environ 50 % en acides gras, avec des atomes de carbone d'une longueur de chaîne supérieure ou égale à 20.

4. Préparation selon l'une quelconque des revendications 1 à 3, **caractérisée par** une teneur en acide béhénique d'un total d'au moins 1 %, de préférence d'au moins 2,5 %, et d'un maximum de 15 %.

5. Préparation selon la revendication 4, **caractérisée en ce qu'**à température ambiante, le mélange de LCT contenant des glycérides d'acide béhénique se présente dans la forme β' de la structure cristalline.

6. Préparation selon l'une quelconque des revendications 1 à 5, **caractérisée en ce que** la teneur en mélange de MCT contenant de l'acide caprique dans la préparation se situe entre 50 % et 65 %.

7. Préparation selon l'une quelconque des revendications 1 à 6, **caractérisée en ce que** la teneur en mélange de MCT contenant de l'acide caprique dans la préparation se situe entre 30 % et 45 %.

8. Préparation selon l'une quelconque des revendications 1 à 7, **caractérisée en ce que** l'indice d'iode dans le mélange de MCT et/ou dans le mélange de LCT et/ou dans l'ensemble de la préparation est inférieur à 30, de préférence inférieur à 2, de manière encore plus préférée, inférieur à 1.

9. Préparation selon l'une quelconque des revendications 1 à 8, **caractérisée en ce qu'**elle est anhydre.

10. Préparation selon l'une quelconque des revendications 1 à 9, **caractérisée en ce qu'**elle ne contient pas d'émulsifiant.
